(19) Europäisches Patentamt
European Patent Office
Office européen des brevets

(11) EP 1 556 339 B1

(12) **EUROPÄISCHE PATENTSCHRIFT**

(45) Veröffentlichungstag und Bekanntmachung des Hinweises auf die Patenterteilung:
**01.09.2010 Patentblatt 2010/35**

(21) Anmeldenummer: **03757879.6**

(22) Anmeldetag: **22.09.2003**

(51) Int Cl.:
***C07C 275/54*** *(2006.01)* ***A61K 31/17*** *(2006.01)*
***A61P 3/10*** *(2006.01)*

(86) Internationale Anmeldenummer:
**PCT/EP2003/010501**

(87) Internationale Veröffentlichungsnummer:
**WO 2004/033416 (22.04.2004 Gazette 2004/17)**

(54) **CARBOXYALKOXY-SUBSTITUIERTE ACYL-CARBOXYPHENYL-HARNSTOFFDERIVATE, VERFAHREN ZU IHRE HERSTELLUNG UND IHRE VERWENDUNG ALS ARZNEIMITTEL**

CARBOXYALKOXY-SUBSTITUTED ACYL-CARBOXYPHENYL-UREA DERIVATIVES, PRODUCTION METHOD AND USE THEREOF AS MEDICINE

DERIVES D'ACYL-CARBOXYPHENYL-UREE A SUBSTITUTION CARBOXYALCOXY, LEUR PROCEDE DE PRODUCTION ET LEUR UTILISATION EN TANT QUE MEDICAMENT

(84) Benannte Vertragsstaaten:
**AT BE BG CH CY CZ DE DK EE ES FI FR GB GR HU IE IT LI LU MC NL PT RO SE SI SK TR**

(30) Priorität: **04.10.2002 DE 10246434**

(43) Veröffentlichungstag der Anmeldung:
**27.07.2005 Patentblatt 2005/30**

(73) Patentinhaber: **Sanofi-Aventis Deutschland GmbH**
**65929 Frankfurt am Main (DE)**

(72) Erfinder:
- **DEFOSSA, Elisabeth**
  **65510 Idstein (DE)**
- **KADEREIT, Dieter**
  **63071 Offenbach (DE)**
- **KLABUNDE, Thomas**
  **65926 Frankfurt am Main (DE)**
- **BURGER, Hans-Joerg**
  **Morristown, NJ 07960 (US)**
- **HERLING, Andreas**
  **65520 Bad Camberg (DE)**
- **WENDT, Karl-Ulrich**
  **65926 Frankfurt am Main (DE)**
- **VON REODERN, Erich**
  **65795 Hattersheim (DE)**
- **SCHOENAFINGER, Karl**
  **63755 Alzenau (DE)**
- **ENHSEN, Alfons**
  **64572 Büttelborn (DE)**

(56) Entgegenhaltungen:
**EP-A- 0 193 249 WO-A-01/94300**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Carboxyalkoxy-substituierte Acyl-carboxyphenyl-hamstoffderivate sowie deren physiologisch verträgliche Salze und physiologisch funktionelle Derivate.

Es sind bereits Acylphenylharnstoffderivate als Antitumormittel und als Antidiabetika im Stand der Technik beschrieben (EP 0 193 249 und WO 01/94300).

Der Erfindung lag die Aufgabe zugrunde, Verbindungen zur Verfügung zu stellen, die eine therapeutisch verwertbare Blutzucker senkende Wirkung entfalten. Insbesonders bestand die Aufgabe darin Verbindungen mit verbesserter Wirkung gegenüber den Verbindungen aus WO 01/94300 zur Verfügung zu stellen.

Die Erfindung betrifft daher Verbindungen der Formel I,

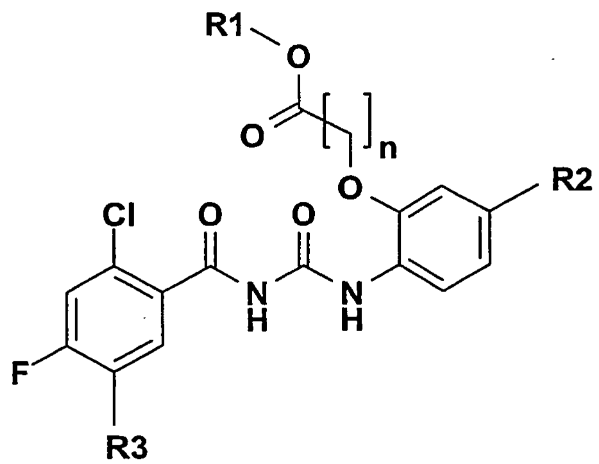

I

worin bedeuten

R1 H, $(C_1\text{-}C_6)$-Alkyl, $(C_0\text{-}C_6)$-Alkyl-Phenyl, wobei der Phenylring bis zu zweifach mit F, Cl, CN, OH, $(C_1\text{-}C_6)$-Alkyl, O-$(C_1\text{-}C_6)$-Alkyl, $CF_3$, $OCF_3$, COOH, COO$(C_1\text{-}C_6)$-Alkyl oder $CONH_2$ substituiert sein kann;

R2 H, $(C_1\text{-}C_6)$-Alkyl, O-$(C_1\text{-}C_6)$-Alkyl, CO-$(C_1\text{-}C_6)$-Alkyl, COO-$(C_1\text{-}C_6)$-Alkyl, $(C_0\text{-}C_6)$-Alkylen-COOH;

R3 H, F, Cl, Br, OH, $CF_3$, $NO_2$, CN, $OCF_3$, O-$(C_1\text{-}C_6)$-Alkyl, $(C_1\text{-}C_6)$-Alkyl;

n 1,2,3,4,5,6,7,8;

sowie deren physiologisch verträgliche Salze.

Bevorzugt sind Verbindungen der Formel I, worin bedeuten

R1 H, $(C_1\text{-}C_6)$-Alkyl;

R2 H, $(C_1\text{-}C_6)$-Alkyl, O-$(C_1\text{-}C_6)$-Alkyl, CO-$(C_1\text{-}C_6)$-Alkyl, COO-$(C_1\text{-}C_6)$-Alkyl, $(C_0\text{-}C_6)$-Alkylen-COOH;

R3 H, F, Cl, Br, OH, $CF_3$, $NO_2$, CN, $OCF_3$, O-$(C_1\text{-}C_6)$-Alkyl, $(C_1\text{-}C_6)$-Alkyl;

n 1, 2, 3, 4, 5, 6, 7, 8;

sowie deren physiologisch verträgliche Salze.

Besonders bevorzugt sind Verbindungen der Formel I, worin bedeuten

R1 H, $(C_1\text{-}C_6)$-Alkyl;

R2 H, COO-$(C_1\text{-}C_6)$-Alkyl, -COOH;

R3 H, F;

n 1, 2, 3, 4;

sowie deren physiologisch verträgliche Salze.

Die Erfindung bezieht sich auf Verbindungen der Formel I, in Form ihrer Racemate, racemischen Mischungen und reinen Enantiomere sowie auf ihre Diastereomere und Mischungen davon.

Die Alkylreste in den Substituenten R1, R2 und R3, können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Die Verbindung(en) der Formel (I) können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Als weitere Wirkstoffe für die Kombinationspräparate sind geeignet:
Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus® (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylharnstoffe, Biguanide, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse

beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, GI 262570, verabreicht.

Bei einer Ausführungsform der Erfindung werden die Verbindungen der Formel I in Kombination mit einem PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US00/11833, PCT/US00/11490, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide, BMS-201038, R-103757, verabreicht.

Bei einer Ausführungsform der Erfindung werden die Verbindungen der Formel I in Kombination mit einem Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705, verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. CI-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.

Bei einer Ausführungsform werden die Verbindungen der Formel I in Kombination mit einem $\alpha$-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., in.:Hormone and Metabolic Research (2001), 33(9), 554-558), NPY-Antagonisten z.B. Naphthalin-1-sulfonsäure {4-[(4-aminoquinazolin-2-ylamino)-methyl]-cyclohexylmethyl}-amid Hydrochlorid (CGP 71683A)), MC4-Agonisten (z.B. 1-Amino-1,2,3,4-tetrahydronaphthalin-2-carbonsäure [2-(3a-benzyl-2-methyl-3-oxo-2,3,3a,4,6,7-hexahydropyrazolo[4,3-c]pyridin-5-yl)-1-(4-chlorophenyl)-2-oxoethyl]-amid; (WO 01/91752)), Orexin-Antagonisten (z.B. 1-(2-Methylbenzoxazol-6-yl)-3-[1,5]naphthyridin-4-yl-harnstoff Hydrochlorid (SB-334867-A)), H3-Agonisten (3-Cyclohexyl-1-(4,4-dimethyl-1,4,6,7-tetrahydro-imidazo[4,5-c]pyridin-5-yl)-propan-1-on Oxalsäuresalz (WO 00 / 63208)); TNF-Agonisten, CRF-Antagonisten (z.B. [2-Methyl-9-(2,4,6-trimethylphenyl)-9H-1,3,9-triazafluoren-4-yl]-dipropylamin (WO 00/66585)), CRF BP-Antagonisten (z.B. Urocortin), Urocortin-Agonisten, β3-Agonisten (z.B. 1-(4-Chlor-3-methansulfonylmethylphenyl)-2-[2-(2,3-dimethyl-1H-indol-6-yloxy)-ethylamino]-ethanol Hydrochlorid (WO 01/83451)), MSH (Melanocyt-stimulierendes Hormon)-Agonisten, CCK-A Agonisten (z.B. {2-[4-(4-Chlor-2,5-dimethoxyphenyl)-5-(2-cyclohexylethyl)-thiazol-2-ylcarbamoyl]-5,7-dimethylindol-1-yl}-essigsäure Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isochinolin-2-carbonsäure-tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),

DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff Fenfluramin oder Dexfenfluramin. Bei noch einer Ausführungsform ist der weitere Wirkstoff Sibutramin.

Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/Caromax®(Zunft H J; et al., Carob pulp preparation for treatment of hypercholesterolemia, ADVANCES IN THERAPY (2001 Sep-Oct), 18(5), 230-6). Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax® kann in einer Zubereitung erfolgen, oder durch getrennte Gabe von Verbindungen der Formel I und Caromax®. Caromax® kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

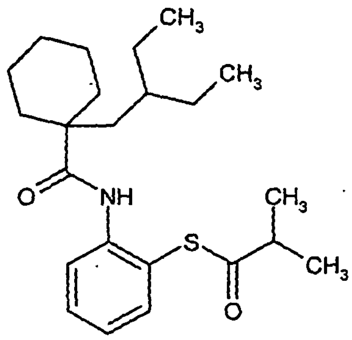

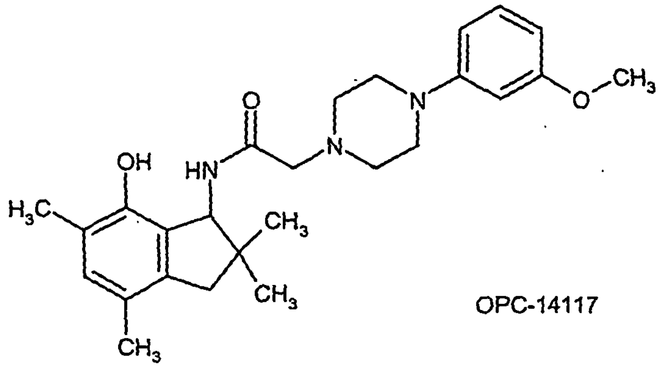

OPC-14117

JTT-705

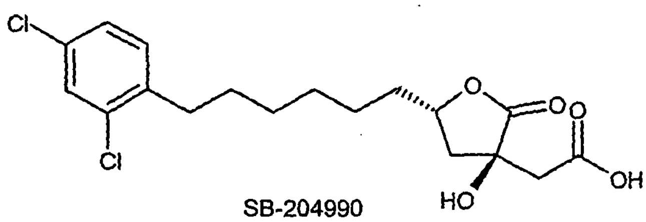

SB-204990

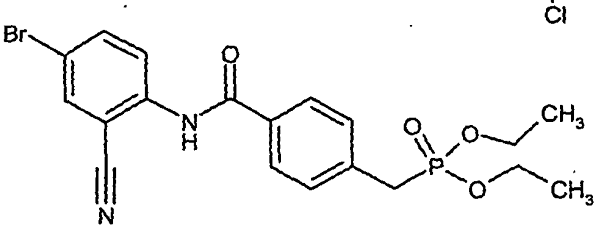

NO-1886

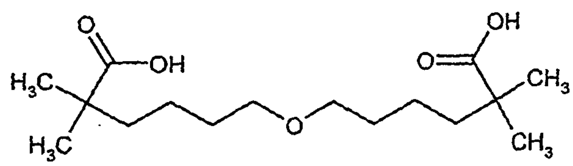

CI-1027

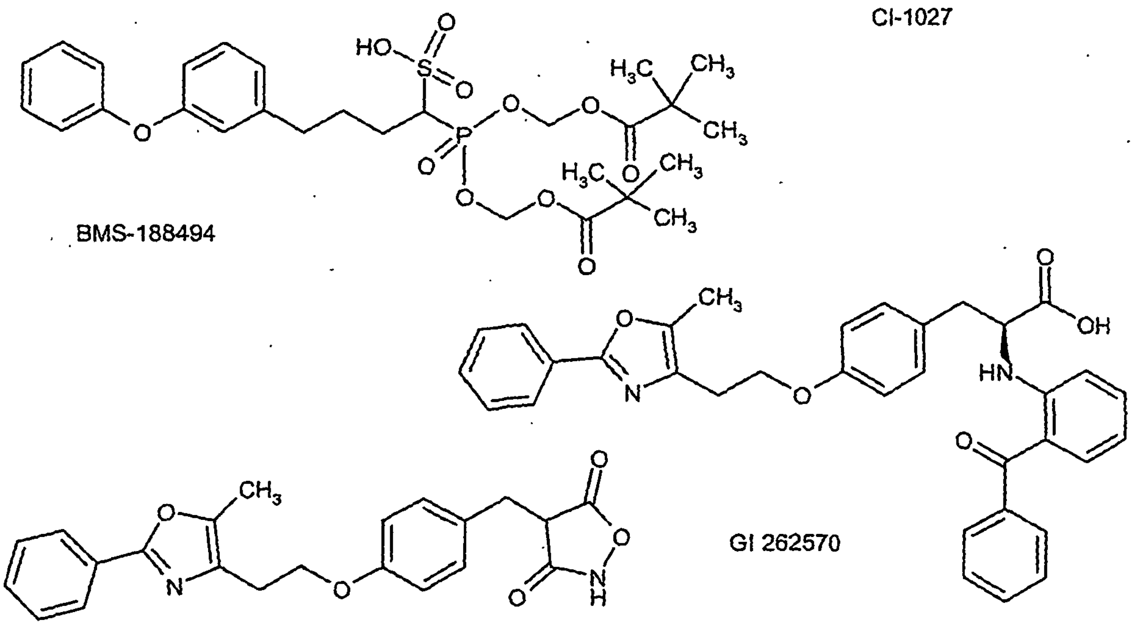

BMS-188494

GI 262570

JTT-501

Die nachfolgend aufgeführten Beispiele dienen zur Erläuterung der Erfindung, ohne diese jedoch einzuschränken. Die

gemessenen Fest-, bzw. Zersetungspunkte (Fp.) wurden nicht korrigiert und sind generell von der Aufheizgeschwindigkeit abhängig.

Tabelle 1: Beispiele

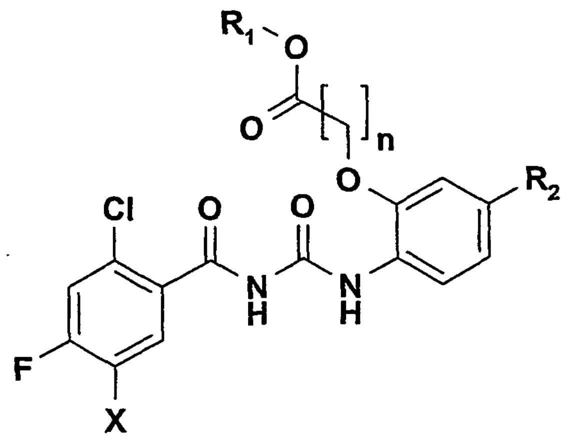

| Beispiel-Nr. | X | R1 | R2 | n | Smp. [°C] | MS |
|---|---|---|---|---|---|---|
| 1 | H | H | H | 1 | 180-181 | ok |
| 2 | H | H | COOH | 1 | 279-281 | ok |
| 3 | F | H | H | 1 | 179-180 | ok |
| 4 | F | Me | COOMe | 1 | 268 | ok |
| 5 | F | H | COOH | 1 | 294-297 | ok |
| 6 | F | H | H | 3 | 168 | ok |
| 7 | F | Me | H | 3 | 228 | ok |
| 8 | F | Me | COOMe | 3 | 209 | ok |
| 9 | F | H | COOH | 3 | 194 | ok |
| 10 | F | Me | COOH | 3 | 227 | ok |
| 11 | H | H | H | 4 | 197 | ok |
| 12 | H | H | COOH | 4 | 253-254 | ok |
| 13 | F | H | H | 4 | 164 | ok |
| 14 | F | H | COOH | 4 | 293-296 | ok |
| 15 | F | Me | COOH | 4 | 220 | ok |

* Unter der Angabe "MS ist ok" wird verstanden, daß ein Massenspektrum gemessen wurde und in diesem der Molpeak (Molmasse + $H^+$) nachgewiesen wurde.

Die Verbindungen der Formel I zeichnen sich durch günstige Wirkungen auf den Lipid- und Kohlenhydratstoffwechsels aus, sie senken insbesondere den Blutzuckerspiegel und sind zur Behandlung von Typ 2 Diabetes, von Insulinresistenz, von Dyslipidämien und des metabolischen Syndroms / Syndrom X geeignet. Weiterhin sind die Verbindungen zur Prophylaxe und Behandlung von arteriosklerotischer Erscheinungen geeignet. Die Verbindungen können allein oder in Kombination mit weiteren Blutzucker senkenden Wirkstoffen eingesetzt werden.

Ferner eignen sich die Verbindungen der Formel I infolge ihrer pharmakologischen Eigenschaften (siehe J. L. Treadway, P. Mendys, D. J. Hoover, Exp. Opin. Invest. Drugs 2001, 10(3), 439-454) als cardioprotektive Arzneimittel zur Infarktprophylaxe und der Infarktbehandlung sowie zur Behandlung der Angina Pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Die Verbindungen können allein oder in Kombination mit weiteren cardioprotectiven bzw. antiarrhythmischen Wirkstoffen eingesetzt werden.

Weiterhin sind die Verbindungen der Formel I auf Grund ihrer pharmakologischen Eigenschaften auch zur Behandlung von Tumorerkrankungen geeignet. In verschiedenen Untersuchungen hierzu konnte gezeigt werden, dass ein direkter Zusammenhang zwischen Glycogenspiegel und verschiedenen Parametern des Tumorwachstums und der Tumorentwicklung besteht (siehe M. Rousset, E. Dussaulx, G. Chevalier, A. Zweibaum, J. Natl. Cancer Inst. 1980, 65 (5), 885-889; K. Yano, S. Ohoshima, Y. Shimizu, T. Moriguchi, H. Katayama, Cancer Letters 1996, 110(1,2), 29-34; L. Skwarski, Z. Namiot, J. Stasiewicz, A. Kemona, M. Kralisz, J. Gorski, Cancer Letters 1998, 127(1,2), 123-128; S. Takahashi, A. Satomi, K. Yano, H. Kawase, T. Tanimizu, Y. Tuji, S. Murakami, R. Hirayama, J. Gastroenterology 1999, 34(4), 474-480). Durch die Manipulation der Menge der freigesetzten Glucose ist dadurch auch eine Verringerung des Tumorwachstums möglich. Die Verbindungen der Formel I können hierzu gegebenenfalls auch in Verbindung mit anderen Antitumormedikamenten eingesetzt werden.

Die Wirksamkeit der Verbindungen wurde wie folgt getestet:

Analyse von Testsubstanzen hinsichtlich Ihrer Potenz die Glykogenolyse in Primärkulturen von Rattenhepatozyten zu hemmen

Hepatozyten wurden aus den Lebem von gefütterten Ratten (Sprague-Dawley oder Wistar, 220-240 g Körpergewicht) mittels einer Standard 2-Stufen-Perfusion zuerst mit Calcium-freier Pufferlösung gefolgt von einer Perfusion mit Kollagenase-haltiger Lösung zur Auflösung des Gewebeverbands isoliert (Seglen et al, 1979). Die Zellen wurden in einem Brutschrank mit 90 % Luftfeuchtigkeit bei 37 °C und einer Atmosphäre mit 5 % $CO_2$ in Luft inkubiert. Die Kultivierung erfolgte üblicherweise mit Williams Medium E ergänzt mit 20 mM Glukose, 0,1 mM Fruktose, 1 μM Dexamethason und 100 nM Insulin. Glykogenolyse wurde durch einen Wechsel des Kulturmediums zu vorgewärmtem mit Carbogen begastem und mit Glukagon 100 nM ergänztem Krebs-Henseleit-Bikarbonat Hepes (20 mM) -Puffer, pH 7,4 induziert (Zeitpunkt 0 min). Die Prüfsubstanzen wurden üblicherweise zum Zeitpunkt 0 min als 100-fache Stammlösung in DMSO zugegeben. Die DMSO Endkonzentration war nicht höher als 1 % (v/v). Die Glukose-Menge im Kulturüberstand wurde nach Zugabe von Glukagon in der Gegenwart bzw. Abwesenheit von Prüfsubstanz durch Entnahme von kleinen Aliquots des Zellkulturüberstands zu den Zeitpunkten 0, 30, 60 und 90 min bestimmt. Die Bestimmung der Glukosekonzentration (mM) erfolgte mit enzymatischen Methoden in einem klinisch-chemischen Analyse Labor von Aventis Pharma Deutschland. Die Glukose-Produktionsrate wurde mittels linearer Regression der Glukosekonzentration zu den Zeitpunkten 30, 60 und 90 min mittels folgender Formel ermittelt:

$$\text{Glukoseproduktionsrate (mM/h)} = \text{mM Glukose (90 min)} - \text{mM Glukose (30 min)}.$$

Prozent Hemmung wurde mittels folgender Formel berechnet:

$$\text{Prozent Hemmung} = 100 \times \left[1 - \frac{\text{Glukoseproduktionsrate in Gegenwart von Prüfsubstanz}}{\text{Glukoseproduktionsrate in Abwesenheit von Prüfsubstanz}}\right]$$

$IC_{50}$-Werte ($IC_{50}$: Die Konzentration (μM) an Prüfsubstanz die eine Reduktion von 50 % der Glukoseproduktionsrate bewirkt) wurden mittels der bei relevanten Konzentrationen an Prüfsubstanz erhaltenen Prozent Hemmungen mit Standard-Kurvenanpassungsverfahren abgeschätzt.

Tabelle 2: Biologische Aktivität

| Beispiel-Nr. | IC50 am Hep. |
|---|---|
| 1 | 1.161 |
| 2 | 1.46 |
| 3 | 0.776 |
| 4 | 1.191 |
| 5 | 0.407 |

(fortgesetzt)

| Beispiel-Nr. | IC50 am Hep. |
|---|---|
| **6** | 0.134 |
| **7** | 0.226 |
| **8** | 0.069 |
| **9** | <0,1 |
| **10** | 0.106 |
| **11** | 0.156 |
| **12** | 0.34 |
| **13** | 0.113 |
| **14** | 0.312 |
| **15** | 0.032 |

Aus der Tabelle ist abzulesen, daß die Verbindungen der Formel I die Glycogenolyse in Rattenhepatocyten hemmen und dadurch die Senkung des Blutzuckerspiegels bewirken.

Es wurden 2 Verbindungen aus WO 01/94300 als Vergleichsbeispiele getestet.

Tabelle 3: Biologische Aktivität der Vergleichsbeispiele

| **Struktur** | **MW** | **$IC_{50}$ am Hep [$\mu$M]** |
|---|---|---|
| Beispiel Nr. 105 aus WO 01/94300 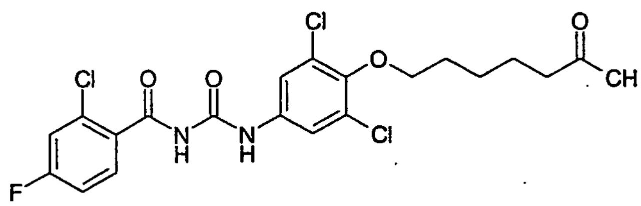 | 491,73 | 17,524 |
| Beispiel Nr. 116 aus WO 01/94300 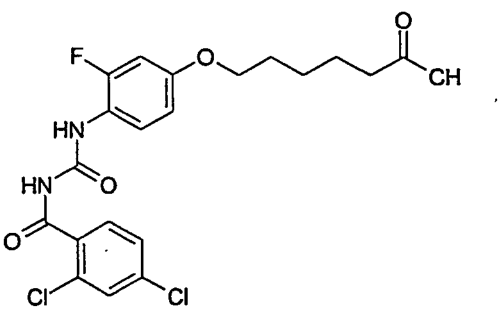 | 457,28 | 13,522 |

Aus der Tabelle ist abzulesen, dass die Verbindung der Formel I eine deutlich erhöhte Wirkung gegenüber den Vergleichsbeispielen aus WO 01/94300 aufweisen. Die Wirkung der erfindungsgemäßen Verbindungen ist 11- bis 548-fach höher.

Nachfolgend wird die Herstellung einiger Beispiele detailliert beschrieben, die übrigen Verbindungen der Formel I wurde analog erhalten:

Experimenteller Teil:

Beispiel 1:
2-[3-(2-Chlor-4-fluorbenzoyl)-ureido]-phenoxyessigsäure

a) 2-Nitrophenoxyessigsäure-tert-butylester

Zu einer Lösung von 1,0 g (7,2 mmol) 2-Nitrophenol in 20 ml Aceton gibt man 2,8 g (14,4 mmol) Bromessigsäure-tert-butylester und 2,6 g (7,9 mmol) Cäsiumkarbonat. Die Suspension wird 48 Stunden zum Rückfluss erhitzt. Anschließend gibt man 50 ml Wasser hinzu und extrahiert zwei mal mit je 50 ml Essigsäureethylester. Die vereinigten organische Phasen werden mit Wasser gewaschen, über $Na_2SO_4$ getrocknet und am Rotationsverdampfer eingeengt. Das Produkt wird ohne Reinigung im nächsten Schritt eingesetzt. Rohausbeute: 2,4 g

b) 2-Aminophenoxyessigsäure-tert-butylester

0,5 g Rohmaterial aus a) werden in Methanol gelöst, mit 0,3 g Raney-Ni versetzt und bei Raumtemperatur mit Wasserstoff hydriert. Die Reaktion wird per LC-MS kontrolliert.

Nach vollständiger Reduktion wird über Celite abgesaugt und mit Methanol nachgewaschen. Das Filtrat wird am Rotationsverdampfer eingeengt und das Produkt ohne Reinigung in Schritt d) eingesetzt. Rohausbeute: 0,34 g

c) 2-Chlor-4-fluorbenzoylisocyanat

2-Chlor-4-fluorbenzamid wurde in Dichlormethan gelöst, mit 1,5 eq. Oxalylchlorid versetzt und 16 Stunden zum Rückfluss erhitzt. Das Reaktionsgemisch wurde am Hochvakuum eingeengt und ohne weitere Reinigung in Stufe d) eingesetzt.

d) 2-[3-(2-Chlor-4-fluorbenzoyl)-ureido]-phenoxyessigsäure-tert-butylester

Zu einer Lösung von 129 mg (0,6 mmol) 2-Aminophenoxyessigsäure-tert-butylester in 5 ml trockenem Acetonitril unter Schutzgasatmosphäre wird bei Raumtemperatur eine Lösung von 230 mg (1,2 mmol) 2-Chlor-4-fluorbenzoylisocyanat in 5 ml Acetonitril zugegeben. Es wird 4 Stunden zum Rückfluss erhitzt und auf Raumtemperatur abgekühlt. Das Lösungsmittel wird am Rotationsverdampfer abdestilliert und der erhaltene Rückstand ohne weitere Aufreinigung in den nächsten Schritt eingesetzt.

Rohausbeute: 0,33 g.

e) 2-[3-(2-Chlor-4-fluorbenzoyl)-ureido]-phenoxyessigsäure-tert-butylester

0,33 g Rohmaterial aus Stufe d) werden in 10 ml Methylenchlorid aufgenommen, mit 10 ml Trifluoressigsäure versetzt und zwei Stunden bei Raumtemperatur gerührt. Die Lösung wird anschließend zweimal nach Zugabe von 3 ml Toluol am Rotationsverdampfer eingeengt und der so erhaltene Rückstand über eine präparative HPLC-Anlage (Säule: Waters Xterra ™MS $C_{18}$, 5 μm, 30x100 mm, Laufmittel: A: $H_2O$ + 0,2 % Trifluoressigsäure, B: Acetonitril, Gradient: 2,5 Minuten 90 % A/ 10 % B bis 17,5 Minuten 10 % A / 90 % B) gereinigt. Man erhält 88 mg (0,23 mmol) des gewünschten

Produktes.

Schmelzpunkt: 180-181 ˚C

Beispiel 3 wurde entsprechend dieser Vorschrift hergestellt.

Beispiel 2:

3-Carboxymethoxy-4-[3-(2-chlor-4-fluor-benzoyl)-ureido]-benzoesäure

a) 3-Methoxycarbonylmethoxy-4-nitrobenzoesäure-methylester

Zu einer Lösung von 0,50 g (2,5 mmol) 3-Hydroxy-4-nitrobenzoesäure-methylester in 10 ml Aceton gibt man 0,77 g (5,1 mmol) Bromessigsäuremethylester und 0,91 g (2,8 mmol) Cäsiumkarbonat. Die Suspension wird 48 Stunden zum Rückfluss erhitzt. Anschließend gibt man 50 ml Wasser hinzu und extrahiert zweimal mit je 50 ml Essigsäureethylester. Die vereinigten organische Phasen werden mit Wasser gewaschen, über $Na_2SO_4$ getrocknet und am Rotationsverdampfer eingeengt. Rohausbeute: 0,64 g

b) 3-Carboxymethoxy-4-[3-(2-chlor-4-fluor-benzoyl)-ureido]-benzoesäure

Das Rohprodukt aus Schritt a) wird analog Beispiel 1b) bis 1d) in 3-Methoxycarbonylmethoxy-4-[3-(2-chlor-4-fluor-benzoyl)-ureido]-benzoesäuremethylester überführt, von dem 50 mg (0,11 mmol) in 5 ml THF gelöst und mit 1 ml Wasser versetzt werden. Dazu werden 27 mg (1,1 mmol) LiOH gegeben und die Reaktion bei Raumtemperatur gerührt bis das Edukt vollständig umgesetzt ist. Die Reaktionskontrolle erfolgt per LC-MS. Nach Abschluß der Reaktion wird mit verdünnter Salzsäure angesäuert und mit zweimal mit je 20 ml Essigsäureethylester extrahiert. Die vereinten organischen Phasen werden über $Na_2SO_4$ getrocknet, filtriert, am Rotationsverdampfer eingeengt und der so erhaltene Rückstand. über eine präparative HPLC-Anlage (Säule: Waters Xterra ™MS $C_{18}$, 5 μm, 30x100 mm, Laufmittel: A: $H_2O$ + 0,2 % Trifluoressigsäure, B: Acetonitril, Gradient: 2,5 Minuten 90 % A /10 % B bis 17,5 Minuten 10 % A / 90 % B) gereinigt. Man erhält 28 mg (0,068 mmol) des gewünschten Produktes.

Schmelzpunkt: 279-281 ˚C

Die Beispiele 4-9 und 11-14 wurden analog dieser Vorschrift hergestellt.

Beispiel 10:
4-[3-(2-Chlor-4,5-difluorbenzoyl)-ureido]-3-(3-methoxycarbonylpropoxy)-benzoesäure

a) 3-Hydroxy-4-nitrobenzoesäure-benzylester
2,0 g (11 mmol) 3-Hydroxy-4-nitrobenzoesäure werden in 25 ml Toluol suspendiert und mit 1,8 g (16 mmol) Benzylalkohol und 0,2 g (1,1 mmol) p-Toluolsulfonsäure versetzt. Unter Verwendung eines Wasserabscheiders erhitzt man die Reaktion zum Rückfluß bis sich kein weiteres Wasser abscheidet. Nach vollständiger Umsetzung wird die Lösung am Rotationsverdampfer eingedampft und das Rohprodukt chromatographisch gereinigt. Ausbeute 1,6 g (5,9 mmol).
b) 4-[3-(2-Chlor-4,5-difluorbenzoyl)-ureido]-3-(3-methoxycarbonylpropoxy)-benzoesäure
Der 3-Hydroxy-4-nitrobenzoesäure-benzylester aus Schritt a) wurde analog Vorschrift 2a) unter Bildung von 3-(3-Methoxycarbonylpropoxy)-4-nitro-benzoesäure-benzylester alkyliert. 450 mg (1,2 mmol) des Benzylesters werden in 20 ml Methanol gelöst mit 13 mg (0,1 mmol) Palladium auf Aktivkohle (10%) versetzt und bei Raumtemperatur hydriert. Die Reaktionskontrolle erfolgt per LC-MS.
Nach vollständiger Reduktion wird über Celite abgesaugt und mit Methanol nachgewaschen. Das Filtrat wird am Rotationsverdampfer eingeengt und das Produkt ohne vorherige Aufreinigung analog Vorschrift 1d) zu 4-[3-(2-Chlor-4,5-difluorbenzoyl)-ureido]-3-(3-methoxycarbonylpropoxy)-benzoesäure umgesetzt. Die abschließende Reinigung erfolgt über präparative HPLC (Säule: Waters Xterra ™MS $C_{18}$, 5 µm, 30x100 mm, Laufmittel: A: $H_2O$ + 0,2 % Trifluoressigsäure, B: Acetonitril, Gradient: 2,5 Minuten 90 % A / 10 % B bis 17,5 Minuten 10 % A / 90 % B). Ausbeute: 0,51 mg (0,11 mmol)
Schmelzpunkt: 227°C

Beispiel 15 wurde analog dieser Vorschrift hergestellt.

## Patentansprüche

**1.** Verbindungen der Formel I,

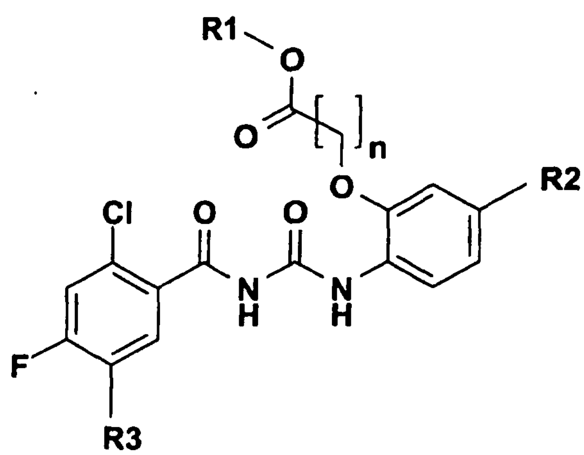

I

worin bedeuten

R1 H, ($C_1$-$C_6$)-Alkyl, ($C_0$-$C_6$)-Alkyl-Phenyl, wobei der Phenylring bis zu zweifach mit F, Cl, CN, OH, ($C_1$-$C_6$)-Alkyl, O-($C_1$-$C_6$)-Alkyl, $CF_3$, $OCF_3$, COOH, COO($C_1$-$C_6$)-Alkyl oder $CONH_2$ substituiert sein kann;
R2 H, ($C_1$-$C_6$)-Alkyl, O-($C_1$-$C_6$)-Alkyl, CO-($C_1$-$C_6$)-Alkyl, COO-($C_1$-$C_6$)-Alkyl, ($C_0$-$C_6$)-Alkylen-COOH;
R3 H, F, Cl, Br, OH, $CF_3$, $NO_2$, CN, $OCF_3$, O-($C_1$-$C_6$)-Alkyl, ($C_1$-$C_6$)-Alkyl;
n 1,2,3,4,5,6,7,8;

sowie deren physiologisch verträgliche Salze.

**2.** Verbindungen der Formel I, gemäß Anspruch 1, **dadurch gekennzeichnet, daß** darin bedeuten

R1 H, ($C_1$-$C_6$)-Alkyl;
R2 H, ($C_1$-$C_6$)-Alkyl, O-($C_1$-$C_6$)-Alkyl, CO-($C_1$-$C_6$)-Alkyl, COO-($C_1$-$C_6$)-Alkyl, ($C_0$-$C_6$)-Alkylen-COOH;
R3 H, F, Cl, Br, OH, $CF_3$, $NO_2$, CN, $OCF_3$, O-($C_1$-$C_6$)-Alkyl, ($C_1$-$C_6$)-Alkyl;
n 1, 2, 3, 4, 5, 6, 7, 8;

sowie deren physiologisch verträgliche Salze.

**3.** Verbindungen der Formel I, gemäß Anspruch 1 oder 2, **dadurch gekennzeichnet, daß** darin bedeuten

R1 H, ($C_1$-$C_6$)-Alkyl;
R2 H, COO-($C_1$-$C_6$)-Alkyl, -COOH;
R3 H, F;
n 1, 2, 3, 4;

sowie deren physiologisch verträgliche Salze.

**4.** Arzneimittel enthaltend eine oder mehrere der Verbindungen gemäß einem oder mehreren der Ansprüche 1 bis 3.

**5.** Arzneimittel enthaltend eine oder mehrere der Verbindungen gemäß einem oder mehreren der Ansprüche 1 bis 3 und mindestens einen weiteren Wirkstoff.

**6.** Arzneimittel, gemäß Anspruch 5, **dadurch gekennzeichnet, daß** es als weiteren Wirkstoff eine oder mehrere Antidiabetika, hypoglykämischen Wirkstoffe, Antiobesics, Anorektika, HMGCoA-Reduktase Inhibitoren, Cholesterinresorptionsinhibitoren, PPAR gamma Agonisten, PPAR alpha Agonisten, PPAR alpha/gamma Agonisten, PPAR delta-Agonisten, Fibrate, MTP-Inhibitoren, Gallensäureresorptionsinhibitoren, CETP-Inhibitoren, polymere Gallensäureadsorber, LDL-Rezeptorinducer, Cholesterin-Absorptionsinhibitoren (Ezetimibe), ACAT-Inhibitoren, Antioxidantien, Lipoprotein-Lipase Inhibitoren, ATP-Citrat-Lyase Inhibitoren, ACC-Inhibitoren, Squalen synthetase inhibitoren, Lipoprotein(a) antagonisten, Lipase Inhibitoren, Insuline, Sulphonylharnstoffe, Biguanide, Glitinide, Thiazolidindione, $\alpha$-Glukosidase-Inhibitoren, Glucagon-Rezeptor-Antagonisten, auf den ATP-abhängigen Kaliumkanal der Betazellen wirkende Wirkstoffe, CART-Agonisten, NPY-Antagonisten, GLP1-Agonisten, GIP-Agonisten, MC4-Agonisten, MCH-Antagonisten, Orexin-Agonisten, H3-Agonisten, TNF-Agonisten, CRF-Agonisten, CRF BP-Antagonisten, Urocortin-Agonisten, $\beta$3-Agonisten, MSH (Melanocyt-stimulierendes Hormon)-Agonisten, CCK-Agonisten, Serotonin-Wiederaufnahme-Inhibitoren, gemischte Serotonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, LXR-Modulatoren, FXR- Modulatoren, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren, TR-$\beta$-Agonisten oder Amphetamine enthält.

**7.** Verwendung der Verbindungen gemäß einem oder mehreren der Ansprüche 1 bis 3 zur Herstellung eines Medikamentes zur Blutzuckersenkung.

**8.** Verwendung der Verbindungen gemäß einem oder mehreren der Ansprüche 1 bis 3 zur Herstellung eines Medikamentes zur Behandlung des Typ II Diabetes.

**9.** Verwendung der Verbindungen gemäß einem oder mehreren der Ansprüche 1 bis 3 zur Herstellung eines Medikamentes zur Behandlung von Lipid- und Kohlenhydratstoffwechselstörungen.

**10.** Verwendung der Verbindungen gemäß einem oder mehreren der Ansprüche 1 bis 3 zur Herstellung eines Medikaments zur Behandlung arteriosklerotischer Erscheinungen.

**11.** Verwendung der Verbindungen gemäß einem oder mehreren der Ansprüche 1 bis 3 zur Herstellung eines Medikaments zur Behandlung von Insulin Resistenz.

**12.** Verfahren zur Herstellung eines Arzneimittels enthaltend eine oder mehrere der Verbindungen gemäß einem oder mehreren der Ansprüche 1 bis 3, **dadurch gekennzeichnet, dass** der Wirkstoff mit einem pharmazeutisch geeigneten Träger vermischt wird und diese Mischung in eine für die Verabreichung geeignete Form gebracht wird.

13. Verwendung der Verbindungen gemäß einem oder mehreren der Ansprüche 1 bis 3 zur Herstellung eines Medikaments zur Behandlung oder Prophylaxe von akuten und chronischen Schäden und Erkrankungen des Herzens, peripherer Organe und Gliedmaßen, die durch Ischämie- oder durch Reperfusionsereignisse verursacht werden.

14. Verwendung der Verbindungen gemäß einem oder mehreren der Ansprüche 1 bis 3 zur Herstellung eines Medikaments zur Behandlung oder Prophylaxe von akuten und chronischen Schäden und Erkrankungen des Herzens, die durch Ischämie- oder durch Reperfusionsereignisse verursacht werden, und zur Behandlung oder Prophylaxe des Herzinfarkts.

15. Verwendung der Verbindungen gemäß einem oder mehreren der Ansprüche 1 bis 3 zur Herstellung eines Medikaments zur Behandlung oder Prophylaxe der Angina Pectoris.

16. Verwendung der Verbindungen gemäß einem oder mehreren der Ansprüche 1 bis 3 zur Herstellung eines Medikaments zur Behandlung oder Prophylaxe von ischämischen Zuständen des Herzens.

17. Verwendung der Verbindungen gemäß einem oder mehreren der Ansprüche 1 bis 3 zur Herstellung eines Medikaments zur Behandlung oder Prophylaxe von ischämischen Zuständen des peripheren und zentralen Nervensystems und des Schlaganfalls.

18. Verwendung der Verbindungen gemäß einem oder mehreren der Ansprüche 1 bis 3 zur Herstellung eines Medikaments zur Behandlung oder Prophylaxe von ischämischen Zuständen peripherer Organe und Gliedmaßen.

19. Verwendung der Verbindungen gemäß einem oder mehreren der Ansprüche 1 bis 3 zur Herstellung eines Medikaments zur Behandlung von Tumorerkrankungen

20. Verwendung der Verbindungen gemäß einem oder mehreren der Ansprüche 1 bis 3 zur Herstellung eines Medikaments zur Behandlung von Tumorerkrankungen, bei denen das Tumorwachstum von der Glycogen Phosphorylase-Aktivität abhängig ist.

21. Verwendung der Verbindungen gemäß einem oder mehreren der Ansprüche 1 bis 3 zur Herstellung eines Medikaments zur Behandlung von Lungen-, Brust- und Darmkrebs.

## Claims

1. A compound of the formula I,

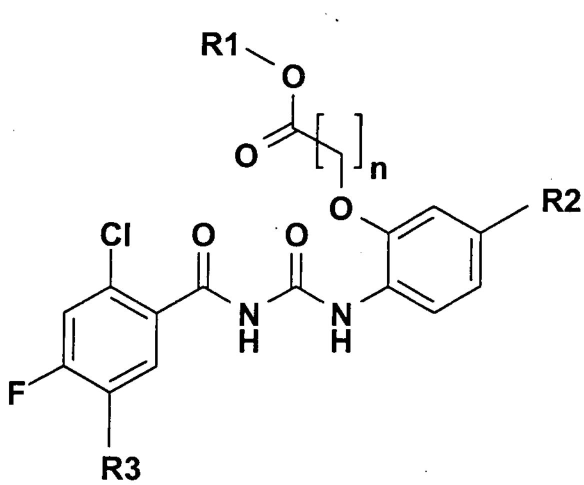

I

in which

R1 is H, $(C_1-C_6)$-alkyl, $(C_0-C_6)$-alkyl-phenyl, where the phenyl ring may be substituted up to twice by F, Cl, CN, OH, $(C_1-C_6)$-alkyl, O- $(C_1-C_6)$-alkyl, $CF_3$, $OCF_3$, COOH, $COO(C_1-C_6)$-alkyl or $CONH_2$;
R2 is H, $(C_1-C_6)$-alkyl, O-$(C_1-C_6)$-alkyl, CO-$(C_1-C_6)$-alkyl, COO-$(C_1-C_6)$- alkyl, $(C_0-C_6)$-alkylene-COOH;
R3 is H, F, Cl, Br, OH, $CF_3$, $NO_2$, CN, $OCF_3$, O-$(C_1-C_6)$-alkyl, $(C_1-C_6)$- alkyl;
n is 1, 2, 3, 4, 5, 6, 7, 8;

and the physiologically tolerated salts thereof.

**2.** A compound of the formula I as claimed in claim 1, where

R1 is H, $(C_1-C_6)$-alkyl;
R2 is H, $(C_1-C_6)$-alkyl, O-$(C_1-C_6)$-alkyl, CO-$(C_1-C_6)$-alkyl, COO-$(C_1-C_6)$- alkyl, $(C_0-C_6)$-alkylene-COOH;
R3 is H, F, Cl, Br, OH, $CF_3$, $NO_2$, CN, $OCF_3$, O-$(C_1-C_6)$-alkyl, $(C_1-C_6)$- alkyl;
n is 1, 2, 3, 4, 5, 6, 7, 8;

and the physiologically tolerated salts thereof.

**3.** A compound of the formula I as claimed in claim 1 or 2, wherein

R1 is H, $(C_1-C_6)$-alkyl;
R2 is H, COO-$(C_1-C_6)$-alkyl, -COOH;
R3 is H, F;
n is 1, 2, 3, 4;

and the physiologically tolerated salts thereof.

**4.** A medicament comprising one or more of the compounds as claimed in one or more of claims 1 to 3.

**5.** A medicament comprising one or more of the compounds as claimed in one or more of claims 1 to 3 and at least one other active ingredient.

**6.** A medicament as claimed in claim 5, wherein the other active ingredient comprises one or more antidiabetics, hypoglycemic active ingredients, antiobesics, anorexia, HMG-CoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, CETP inhibitors, polymeric bile acid adsorbents, LDL receptor inducers, cholesterol absorption inhibitors (ezetimibe), ACAT inhibitors, antioxidants, lipoprotein lipase inhibitors, ATP-citrate lyase inhibitors, ACC inhibitors, squalene synthetase inhibitors, lipoprotein(a) antagonists, lipase inhibitors, insulins, sulfonylureas, biguanides, glitinides, thiazolidinediones, $\alpha$-glucosidase inhibitors, glucagon-receptor antagonists, active ingredients which act on the ATP-dependent potassium channel of the beta cells, CART agonists, NPY agonists, GLP1 agonists, GIP agonists, MC4 agonists, MCH antagonists, orexin agonists, H3 agonists, TNF agonists, CRF agonists, CRF BP antagonists, urocortin agonists, $\beta$3 agonists, MSH (melanocytestimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, LXR modulators, FXR modulators, uncoupling protein 2 or 3 modulators, leptin agonists, DA agonists (bromocriptine, Doprexin), lipase/amylase inhibitors, PPAR modulators, RXR modulators, TR-$\beta$ agonists or amphetamines.

**7.** The use of the compounds as claimed in one or more of claims 1 to 3 for producing a medicament for reducing blood glucose.

**8.** The use of the compounds as claimed in one or more of claims 1 to 3 for producing a medicament for the treatment of type II diabetes.

**9.** The use of the compounds as claimed in one or more of claims 1 to 3 for producing a medicament for the treatment of disturbances of lipid and carbohydrate metabolism.

**10.** The use of the compounds as claimed in one or more of claims 1 to 3 for producing a medicament for the treatment of arteriosclerotic manifestations.

**11.** The use of the compounds as claimed in one or more of claims 1 to 3 for producing a medicament for the treatment of insulin resistance.

**12.** A process for producing a medicament comprising one or more of the compounds as claimed in one or more of claims 1 to 3, which comprises mixing the active ingredient with a pharmaceutically suitable carrier and converting this mixture into a form suitable for administration.

**13.** The use of the compounds as claimed in one or more of claims 1 to 3 for producing a medicament for the treatment or prophylaxis of acute and chronic damage and disorders of the heart, peripheral organs and limbs which are caused by ischemic events or by reperfusion events.

**14.** The use of the compounds as claimed in one or more of claims 1 to 3 for producing a medicament for the treatment or prophylaxis of acute and chronic damage and disorders of the heart which are caused by ischemic events or by reperfusion events, and for the treatment or prophylaxis of myocardial infarction.

**15.** The use of the compounds as claimed in one or more of claims 1 to 3 for producing a medicament for the treatment or prophylaxis of angina pectoris.

**16.** The use of the compounds as claimed in one or more of claims 1 to 3 for producing a medicament for the treatment or prophylaxis of ischemic states of the heart.

**17.** The use of the compounds as claimed in one or more of claims 1 to 3 for producing a medicament for the treatment or prophylaxis of ischemic states of the peripheral and central nervous system and of stroke.

**18.** The use of the compounds as claimed in one or more of claims 1 to 3 for producing a medicament for the treatment or prophylaxis of ischemic states of the peripheral organs and limbs.

**19.** The use of the compounds as claimed in one or more of claims 1 to 3 for producing a medicament for the treatment of oncoses.

**20.** The use of the compounds as claimed in one or more of claims 1 to 3 for producing a medicament for the treatment of oncoses in which the tumor growth is dependent on glycogen phosphorylase activity.

**21.** The use of the compounds as claimed in one or more of claims 1 to 3 for producing a medicament for the treatment of cancer of the lung, breast and bowel.

**Revendications**

**1.** Composés de formule I,

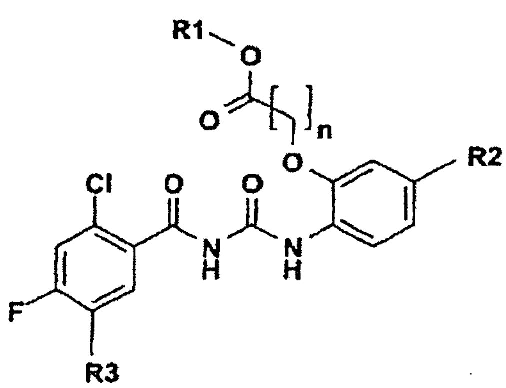

*I*

où

R1 signifie H, $(C_1-C_6)$-alkyle, $(C_0-C_6)$-alkyl-phényle, où le cycle phényle peut être jusqu'à disubstitué par F, Cl, CN, OH, $(C_1-C_6)$-alkyle, O-$(C_1-C_6)$-alkyle, $CF_3$, $OCF_3$, COOH, COO$(C_1-C_6)$-alkyle ou $CONH_2$ ;
R2 signifie H, $(C_1-C_6)$-alkyle, O-$(C_1-C_6)$-alkyle, CO- $(C_1-C_6)$-alkyle, COO-$(C_1-C_6)$-alkyle, $(C_0-C_6)$- alkylène-COOH ;
R3 signifie H, F, Cl, Br, OH, $CF_3$, $NO_2$, CN, $OCF_3$, O- $(C_1-C_6)$-alkyle, $(C_1-C_6)$-alkyle;
n vaut 1, 2, 3, 4, 5, 6, 7, 8 ;

ainsi que leurs sels physiologiquement acceptables.

**2.** Composés de formule I, selon la revendication 1, **caractérisés en ce que**

R1 signifie H, $(C_1-C_6)$-alkyle ;
R2 signifie H, $(C_1-C_6)$-alkyle, O-$(C_1-C_6)$-alkyle, CO- $(C_1-C_6)$-alkyle, COO-$(C_1-C_6)$-alkyle, $(C_0-C_6)$- alkylène-COOH ;
R3 signifie H, F, Cl, Br, OH, $CF_3$, $NO_2$, CN, $OCF_3$, O- $(C_1-C_6)$-alkyle, $(C_1-C_6)$-alkyle ;
n vaut 1, 2, 3, 4, 5, 6, 7, 8 ;

ainsi que leurs sels physiologiquement acceptables.

**3.** Composés de formule I, selon la revendication 1 ou 2, **caractérisés en ce que**

R1 signifie H, $(C_1-C_6)$-alkyle ;
R2 signifie H, COO-$(C_1-C_6)$-alkyle, -COOH ;
R3 signifie H, F ;
n vaut 1, 2, 3, 4 ;

ainsi que leurs sels physiologiquement acceptables.

**4.** Médicament contenant un ou plusieurs des composés selon l'une ou plusieurs des revendications 1 à 3.

**5.** Médicament contenant un ou plusieurs des composés selon l'une ou plusieurs des revendications 1 à 3 et au moins une autre substance active.

**6.** Médicament selon la revendication 5, **caractérisé en ce qu'**il contient comme autre substance active un(e) ou plusieurs antidiabétiques, substances actives hypoglycémiques, agents d'anti-obésité, anorexigènes, inhibiteurs de la HMGCoA-réductase, inhibiteurs de la résorption de cholestérol, agonistes de PPAR gamma, agonistes de PPAR alpha, agonistes de PPAR alpha/gamma, agonistes de PPAR delta, fibrates, inhibiteurs de MTP, inhibiteurs de la résorption de l'acide biliaire, inhibiteurs de CETP, adsorbants polymères de l'acide biliaire, inducteurs de récepteurs de LDL, inhibiteurs de l'absorption du cholestérol (Ezetimibe), inhibiteurs d'ACAT, antioxydants, inhibiteurs de la lipoprotéine-lipase, inhibiteurs de l'ATP-citrate lyase, inhibiteurs d'ACC, inhibiteurs de la squalène-synthétase, antagonistes de la lipoprotéine (a), inhibiteurs de lipase, insulines, sulfonylurées, biguanides, glitinides, thiazolidinediones, inhibiteurs de l'$\alpha$-glucosidase, antagonistes du récepteur de glucagon, substances actives agissant sur le canal potassique dépendant d'ATP des cellules bêta, agonistes de CART, antagonistes de NPY, agonistes de GLP1, agonistes de GIP, agonistes de MC4, antagonistes de MCH, agonistes d'orexine, agonistes de H3, agonistes de TNF, agonistes de CRF, antagonistes de CRF BP, agonistes d'urocortine, agonistes de $\beta$3, agonistes de MSH (hormone stimulant les mélanocytes), agonistes de CCK, inhibiteurs de la résorption de la sérotonine, composés sérotoninergiques et noradrénergiques mixtes, agonistes de 5HT, agonistes de bombésine, agonistes de galanine, hormones de croissance, composés libérant l'hormone de croissance, agonistes de TRH, modulateurs de LXR, modulateurs de FXR, modulateurs 2 ou 3 de la protéine découplante, agonistes de leptine, agonistes de DA (Bromocriptine, Doprexine), inhibiteurs de lipase/amylase, modulateurs de PPAR, modulateurs de RXR, agonistes de TR-15 ou amphétamines.

**7.** Utilisation des composés selon l'une ou plusieurs des revendications 1 à 3 pour la préparation d'un médicament destiné à abaisser la glycémie.

**8.** Utilisation des composés selon l'une ou plusieurs des revendications 1 à 3 pour la préparation d'un médicament destiné au traitement du diabète de type II.

**9.** Utilisation des composés selon l'une ou plusieurs des revendications 1 à 3 pour la préparation d'un médicament destiné au traitement des troubles du métabolisme lipidique et des hydrates de carbone.

**10.** Utilisation des composés selon l'une ou plusieurs des revendications 1 à 3 pour la préparation d'un médicament destiné au traitement de phénomènes artérioscléreux.

**11.** Utilisation des composés selon l'une ou plusieurs des revendications 1 à 3 pour la préparation d'un médicament destiné au traitement de la résistance à l'insuline.

**12.** Procédé pour la préparation d'un médicament contenant un ou plusieurs des composés selon l'une ou plusieurs des revendications 1 à 3, **caractérisé en ce que** la substance active est mélangée avec un support pharmaceutiquement approprié et ce mélange est amené dans une forme appropriée pour l'administration.

**13.** Utilisation des composés selon l'une ou plusieurs des revendications 1 à 3 pour la préparation d'un médicament destiné au traitement ou à la prophylaxie de dommages aigus et chroniques et de maladies aiguës et chroniques du coeur, des organes périphériques et des membres, qui sont provoqués par des phénomènes d'ischémie ou de reperfusion.

**14.** Utilisation des composés selon l'une ou plusieurs des revendications 1 à 3 pour la préparation d'un médicament destiné au traitement ou à la prophylaxie de dommages aigus et chroniques et de maladies aiguës et chroniques du coeur qui sont provoqués par des phénomènes d'ischémie ou de reperfusion et au traitement ou à la prophylaxie de l'infarctus du myocarde.

**15.** Utilisation des composés selon l'une ou plusieurs des revendications 1 à 3 pour la préparation d'un médicament destiné au traitement ou à la prophylaxie de l'angine de poitrine.

**16.** Utilisation des composés selon l'une ou plusieurs des revendications 1 à 3 pour la préparation d'un médicament destiné au traitement ou à la prophylaxie d'états ischémiques du coeur.

**17.** Utilisation des composés selon l'une ou plusieurs des revendications 1 à 3 pour la préparation d'un médicament destiné au traitement ou à la prophylaxie d'états ischémiques du système nerveux périphérique et central et de l'attaque.

**18.** Utilisation des composés selon l'une ou plusieurs des revendications 1 à 3 pour la préparation d'un médicament destiné au traitement ou à la prophylaxie d'états ischémiques d'organes périphériques et des membres.

**19.** Utilisation des composés selon l'une ou plusieurs des revendications 1 à 3 pour la préparation d'un médicament destiné au traitement de maladies tumorales.

**20.** Utilisation des composés selon l'une ou plusieurs des revendications 1 à 3 pour la préparation d'un médicament destiné au traitement de maladies tumorales dans lesquelles la croissance tumorale dépend de l'activité de la glycogène phosphorylase.

**21.** Utilisation des composés selon l'une ou plusieurs des revendications 1 à 3 pour la préparation d'un médicament destiné au traitement de du cancer des poumons, du sein et de l'intestin.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0193249 A
- WO 0194300 A
- US 6221633 B
- WO 9808871 A
- WO 9726265 A
- WO 9903861 A
- US 0011833 W
- US 0011490 W
- DE 10142734
- US 6245744 B
- US 6221897 B
- US 6342512 B
- WO 9741097 A
- WO 0191752 A
- WO 0063208 A
- WO 0066585 A
- WO 0183451 A
- WO 9915525 A
- WO 0071549 A
- WO 0109111 A
- WO 0185695 A
- EP 0462884 A
- WO 0040569 A
- WO 0078312 A


### In der Beschreibung aufgeführte Nicht-Patentliteratur


- **H. Okada et al.** *Chem. Pharm. Bull.,* 1994, vol. 42, 57-61
- **Asakawa, A et al.** Cocaine-amphetamine-regulated transcript influences energy metabolism, anxiety and gastric emptying in mice. *Hormone and Metabolic Research,* 2001, vol. 33 (9), 554-558
- **Lee, Daniel W. ; Leinung, Matthew C. ; Rozhavskaya-Arena, Marina ; Grasso, Patricia.** Leptin agonists as a potential approach to the treatment of obesity. *Drugs of the Future,* 2001, vol. 26 (9), 873-881
- **Salvador, Javier ; Gomez-Ambrosi, Javier ; Fruhbeck, Gema.** Perspectives in the therapeutic use of leptin. *Expert Opinion on Pharmacotherapy,* 2001, vol. 2 (10), 1615-1622
- **Zunft H J et al.** Carob pulp preparation for treatment of hypercholesterolemia. *ADVANCES IN THERAPY,* September 2001, vol. 18 (5), 230-6
- **J. L. Treadway ; P. Mendys ; D. J. Hoover.** *Exp. Opin. Invest. Drugs,* 2001, vol. 10 (3), 439-454
- **M. Rousset ; E. Dussaulx ; G. Chevalier ; A. Zweibaum.** *J. Natl. Cancer Inst.,* 1980, vol. 65 (5), 885-889
- **K. Yano ; S. Ohoshima ; Y. Shimizu ; T. Moriguchi ; H. Katayama.** *Cancer Letters,* 1996, vol. 110 (1,2), 29-34
- **L. Skwarski ; Z. Namiot ; J. Stasiewicz ; A. Kemona ; M. Kralisz ; J. Gorski.** *Cancer Letters,* 1998, vol. 127 (1,2), 123-128
- **S. Takahashi ; A. Satomi ; K. Yano ; H. Kawase ; T. Tanimizu ; Y. Tuji ; S. Murakami ; R. Hirayama.** *J. Gastroenterology,* 1999, vol. 34 (4), 474-480